# EUROPÄISCHE PATENTSCHRIFT

(11) **EP 3 388 038 B1**
(45) Veröffentlichungstag und Bekanntmachung des Hinweises auf die Patenterteilung: **12.02.2025**
(21) Anmeldenummer: 18166513.4
(22) Anmeldetag: 10.04.2018
(51) Int. Cl.: A61B 5/024, G08B 5/36, G08B 21/04, A61F 13/02, A61B 50/20, A61B 5/00, A61B 5/11, G08B 21/02, G16H 40/67

(54) **HAUTAUFLAGE, ASSISTENZSYSTEM, SET**
SKIN PATCH, ASSISTANCE SYSTEM, SET
COMPRESSE, SYSTÈME D'AIDE, KIT

(30) Priorität: 11.04.2017 DE 102017107864
(43) Veröffentlichungstag der Anmeldung: 17.10.2018
(73) Patentinhaber: Diakoneo KdöR, 91564 Neuendettelsau (DE)
(72) Erfinder: Besser, Jürgen, 90587 Tuchenbach (DE); Zerth, Jürgen, 90763 Fürth (DE)
(74) Vertreter: Bobbert & Partner Patentanwälte PartmbB

(56) Entgegenhaltungen:
- DE-A1- 3 118 232
- US-A1- 2007 027 388
- US-A1- 2015 351 690
- US-A1- 2016 310 663

## Beschreibung

Die vorliegende Erfindung betrifft ein System umfassend eine Hautauflage und ein Assistenzsystem gemäß Anspruch 1.

Aus der Praxis sind Pflaster bekannt, die auf die Haut eines Trägers lösbar aufgeklebt werden.

US2007/027388, US2015/351690 und US2016/310663 offenbaren Hautauflagen mit Assistenzsystemen.

Eine Aufgabe der vorliegenden Erfindung ist es, ein System umfassend eine Hautauflage und ein Assistenzsystem vorzuschlagen.

Die erfindungsgemäße Aufgabe wird durch ein System mit den Merkmalen des Anspruchs 1 gelöst.

Die vorliegende Erfindung betrifft eine Hautauflage mit einem flächigen Trägermaterial. Die erfindungsgemäße Hautauflage und/oder das Trägermaterial umfassen eine im Gebrauch der Haut eines Trägers der Hautauflage zugewandte Seite sowie eine der Haut abgewandte Seite. Das Trägermaterial ist auf seiner der Haut zugewandten Seite zumindest bereichsweise mit einer Klebeschicht versehen, mit welcher die Hautauflage auf der Haut des Trägers fixiert werden kann.

Die erfindungsgemäße Hautauflage weist wenigstens einen ersten Taschenabschnitt auf, welcher von der der Haut abgewandten Seite zugänglich ist, also von dieser Seite z. B. geöffnet, beladen oder befüllt, verschlossen usw. werden kann.

Der Taschenabschnitt kann, insbesondere wiederholend, sowohl geöffnet als auch geschlossen oder verschlossen werden. Er dient der Aufnahme einer Komponente oder weist eine solche in seinem Inneren auf.

Das Assistenzsystem umfasst mindestens einen Prozessor und einen Speicher, wobei der Prozessor geeignet und/oder konfiguriert ist, in einem Speicher abgelegte Befehle auszuführen. Zudem umfasst das Assistenzsystem eine Stromversorgungseinheit und eine Kommunikationseinheit.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Erfindungsgemäße Ausführungsformen können eines oder mehrere der vorstehend oder im Folgenden genannten Merkmale aufweisen. Dabei können die hierin genannten Merkmale in beliebiger Kombination Gegenstand von erfindungsgemäßen Ausführungsformen sein, sofern der Fachmann eine konkrete Kombination nicht als technisch unmöglich erkennt.

Erfindungsgemäße Ausführungsformen sind ferner Gegenstand der Unteransprüche und Ausführungsformen.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wann immer hierin von einer Ausführungsform die Rede ist, so handelt es sich um eine beispielhafte, erfindungsgemäße Ausführungsform.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs beziehen.

In manchen Ausführungsformen umfasst die Hautauflage wenigstens einen zweiten Taschenabschnitt und/oder wenigstens eine zusätzliche Kammer. Diese können zum Aufnehmen eines Gegenstands in ihrem Inneren vorgesehen sein oder weisen einen Gegenstand auf. Der Gegenstand kann ein mehrfach verwendeter oder verwendbarer Gegenstand sein.

In einigen Ausführungsformen ist wenigstens ein Element aus der Gruppe, welche aus erstem Taschenabschnitt, zweitem Taschenabschnitt und zusätzlicher Kammer besteht, an seiner Unterseite mittels des Trägermaterials und an seiner Oberseite von einem Abdeckmaterial begrenzt. Optional sind mehrere Elemente aus vorstehender Gruppe mittels desselben Trägermaterials und/oder desselben Abdeckmaterials begrenzt.

In einigen Ausführungsformen ist das Abdeckmaterial beispielsweise mittels einer Klebeschicht, eines Klettabschnitts und/oder eines Nahtabschnitts mit der der Haut abgewandten Seite des Trägermaterials verbunden.

In manchen Ausführungsformen entspricht dabei das Material des Abdeckmaterials dem Material des Trägermaterials.

In einigen Ausführungsformen weist wenigstens ein Element aus der Gruppe der Hautauflage, welche aus erstem Taschenabschnitt, zweitem Taschenabschnitt und zusätzlicher Kammer besteht, ein Verschlussmittel auf. Ein solches Verschlussmittel kann optional eine Lasche sein, welche einen Einsteckabschnitt, Klettabschnitt und/oder Reißverschlussabschnitt aufweist. Optional ist das Verschlussmittel das Abdeckmaterial, das z. B. mittels Klettverschluss verbunden ist, etwa am Trägermaterial, und dabei ein Inneres oder ein Volumen bildet.

In einigen Ausführungsformen bestehen das Trägermaterial und/oder das Abdeckmaterial aus einem Nonwoven-Material, z. B. aus einem PE-Vlies, oder weist ein solches auf.

In bestimmten Ausführungsformen ist das Trägermaterial eine wasserundurchlässige mikroporöse Kunststoffträgerschicht oder weist eine solche auf.

In bestimmten Ausführungsformen kann das Trägermaterial in seinem inneren Bereich wenigstens eine Öffnung haben und/oder die Form eines umlaufenden Rahmens haben. Vorteilhafterweise kann so eine Materialeinsparung erreicht werden, indem Trägermaterial eingespart wird.

In bestimmten Ausführungsformen kann die Klebeschicht eine Fläche umschreiben und somit in einer Mitte also nicht vorliegen. Dies erlaubt es vorteilhaft, dass weniger Klebematerial mit der Haut des Trägers der Hautauflage in Verbindung kommt. Reaktionen auf das Klebematerial können somit verringert oder vorgebeugt werden. Zudem ist weniger Klebefläche zu entfernen, soll die Hautauflage einmal von der Haut entfernt werden. Letzteres hilft, Zeit zum Lösen der Hautauflage von der Haut einzusparen. Zudem ist die Belastung für den Träger geringer.

In bestimmten Ausführungsformen weist die Hautauflage einen Klettverschluss und/oder ein Abdeckmaterial zwischen dem Trägermaterial einerseits und einem Element aus der Gruppe bestehend aus erstem Taschenabschnitt, zweitem Taschenabschnitt und zusätzlicher Kammer andererseits auf.

In bestimmten Ausführungsformen umfasst die Klebeschicht einen, vorzugsweise druckempfindlichen, Silikonkleber. Druckempfindliche Silikonkleber sind u. a. von Ulman und Thomas in "Silicone Pressure sensitive adhesives for healthcare applications", Advances in Pressure sensitive technology-2 (1995), S. 133-157, beschrieben. Silikonkleber sind vergleichsweise hautverträglich.

In bestimmten Ausführungsformen ist die Klebeschicht auf der der Haut zugewandten Seite vollflächig aufgebracht. In anderen ist sie nur abschnittsweise aufgebracht, etwa in Mustern. Durch einen Auftrag nur in Mustern kann insbesondere die Wasserdampfdurchlässigkeit der Hautauflage erhöht werden. Letzteres kann für den Träger angenehmer sein.

In bestimmten Ausführungsformen ist die Klebkraft des Silikonklebers ca. 0,8 N/25 mm, insbesondere 0,85 bis 0,95 N/25 mm und besonders bevorzugt 0,92 N/25 mm, wobei die Klebkraft nach dem Deutschen Arzneimittelbuch DAB 10 von 1996 mit einem Abzugswinkel von 180° gegenüber rostfreiem Stahl gemessen wurde.

In einigen Ausführungsformen ist das Trägermaterial ein streifenförmiges oder ringförmiges Material mit einer Mittelaussparung.

In manchen Ausführungsformen ist die hautzugewandte Seite des Klebers mit einer im Bereich von 60 bis 150°C hitzestabilen Schutzschicht versehen.

In bestimmten Ausführungsformen weist die Klebeschicht eine ringförmige oder anderweitig geschlossene Grundform auf, die jedoch segmentartig unterbrochen ist, so dass sich eine rippenartige Verbindung zwischen der Haut und dem Trägermaterial ergibt.

Hierdurch kann über das Trägermaterial hinweg die Wasserdampfdurchlässigkeit auch im Bereich des Trägermaterials weiter erhöht werden.

In einigen Ausführungsformen ist wenigstens ein Element aus der Gruppe bestehend aus erstem Taschenabschnitt, zweitem Taschenabschnitt und zusätzlicher Kammer verschließbar, öffenbar, und/oder dient der Aufnahme einer elektronischen Einheit, z. B. eines Assistenzsystems oder einer Komponente hiervon, z. B. einer Stromversorgungseinheit.

In manchen Ausführungsformen ist die Hautauflage kein Wundpflaster.

In manchen Ausführungsformen weist die Hautauflage kein Wundkissen auf, insbesondere keinen Quellkörper, kein superabsorbierendes Material und/oder kein Wundkissen aus einem Sandwich aus zwei Polypropylengestricken und einem dazwischen angeordneten Faser/SAP-Gemisch.

In einigen Ausführungsformen sind Kommunikationseinheit, Stromversorgungseinheit und/oder Satellitenkommunikationseinheit separat ausgeführt und funktionsverbunden. In anderen Ausführungsformen können ein oder mehrere Einheiten und/oder deren Teile jedoch physisch als ein zusammenhängendes Element ausgeführt sein, beispielsweise kann der Prozessor mit dem Speicher ein zusammenhängendes Element bilden. Ebenso können in einigen Ausführungsformen eine oder mehrere Einheiten aus mehreren physisch getrennten Elementen bestehen, beispielsweise kann eine Kommunikationseinheit in mehreren getrennten Elementen ausgeführt sein, die beispielsweise unterschiedliche Arten von Funkkommunikation ermöglichen.

In einigen Ausführungsformen ist die Kommunikationseinheit, der Speicher und der Prozessor sowie optional die Stromversorgungseinheit physisch als ein zusammenhängendes Element ausgeführt. Insbesondere umfasst die Kommunikationseinheit in diesem Fall eine Satellitennavigationseinheit und eine Mobilfunkeinheit.

Die Stromversorgungseinheit umfasst in einigen Ausführungsformen mindestens eine Batterie, die vorzugsweise wiederaufladbar (wiederaufladbare Batterien werden hierin auch als Akku bezeichnet) und/oder austauschbar ist.

In einigen Ausführungsformen umfasst die Stromversorgungseinheit mindestens einen Kondensator zur Stromversorgung (z.B. mindestens einen Superkondensator).

In einigen Ausführungsformen umfasst die Stromversorgungseinheit eine Ladeeinheit zum Aufladen eines Akkus. Die Ladeeinheit umfasst in einigen Ausführungsformen einen Anschluss für eine externe Spannungsquelle, z.B. eine Niedervolt-Gleichspannungsquelle.

In einigen Ausführungsformen ist die Stromversorgungseinheit geeignet und/oder konfiguriert für induktives Laden. Dazu ist die Stromversorgungseinheit vorzugsweise mit einer Spule ausgestattet, in die durch ein induktives Ladegerät Strom zum Aufladen des Akkus und/oder des Kondensators induziert werden kann (z.B. gemäß dem Standard Qi).

In einigen Ausführungsformen kann der Akku und/oder der Kondensator zumindest teilweise durch Energy Harvesting aufgeladen werden. Dabei wird Energie aus der Umwelt aufgenommen und daraus Elektrizität erzeugt. Die Energie kann dabei z.B. kinetische Energie (z.B. aus Vibrationen, lineare und/oder Rotationsbewegungen, Gewichtsverlagerungen), Temperaturunterschiede (z.B. Körpertemperatur gegenüber Umgebungstemperatur) und/oder Photonenenergie (z.B. Sonneneinstrahlung) sein. Dazu kann die Stromversorgungseinheit mindestens ein Peltier-, Piezo- und/oder Solarelement und/oder einen elektromagnetischen Linear- und/oder Rotationsgenerator aufweisen.

Die Kommunikationseinheit umfasst optional einen Empfänger und einen Sender für Signale, insbesondere elektromagnetische Wellen, insbesondere im Bereich der Hochfrequenz. Hochfrequenz bezeichnet elektromagnetische Wellen zwischen 30 kHz und 300 GHz.

Die Kommunikationseinheit ist dazu geeignet und/oder konfiguriert, Informationen zu senden und/oder zu empfangen.

Die Kommunikationseinheit kann ein Mobilfunkmodul umfassen, das entsprechend bekannter Mobilfunkstandards Daten übertragen und/oder Telefonanrufe initiieren und empfangen kann (z.B. unter Verwendung der Mobilfunktechnologien GSM, GPRS, EDGE, UMTS und/oder LTE).

Alternativ oder zusätzlich kann die Kommunikationseinheit ein oder mehrere Funkmodule für Kurzstreckenfunk (z.B. entsprechend dem Standard Bluetooth (BT)), zur Verbindung mit einem Funknetzwerk (z.B. WiFi) und/oder für weitere Funktechniken und -standards (z.B. Z-Wave, Zigbee), insbesondere Niedrigenergiefunktechniken (z.B. LoRa, LoRaWAN, SigFox, NB-Fi) umfassen. Die für die Kommunikation verwendeten Frequenzen liegen dabei in einigen Ausführungsformen in den zugelassenen ISM-Bändern.

In einigen Ausführungsformen ist die Kommunikationseinheit dazu geeignet und/oder konfiguriert, autonom oder in Zusammenwirkung mit dem Prozessor eine Positionsbestimmung basierend auf ortsfesten Sendern von elektromagnetischer Hochfrequenz vorzunehmen. Derartige Sender können beispielsweise sogenannte Beacons sein, die z.B. periodisch eine eindeutige Kennung übermitteln, z.B. in Verbindung mit einer Uhrzeit. Aus der empfangenen Kennung kann die Kommunikationseinheit bei entsprechender Ausführung (ggf. in Zusammenwirkung mit dem Prozessor) eine Position bestimmen. Zur genaueren Positionsbestimmung kann dabei die Kommunikationseinheit in einigen Ausführungsformen die Signalstärke von einem oder mehreren empfangenen Beacons auswerten.

In einigen Ausführungsformen ist die Kommunikationseinheit in der Lage, die Position anhand von Beacons auszuwerten, die einen Bluetooth-Standard verwenden (BT-Beacons).

In einigen Ausführungsformen geschieht die Positionsbestimmung rein passiv, d.h. die Kommunikationseinheit empfängt zur Positionsbestimmung lediglich Signale und versendet zu diesem Zweck keine Signale.

In alternativen Ausführungsformen kann die Kommunikationseinheit zur Positionsbestimmung sowohl Signale empfangen als auch Signale aussenden. Beispielsweise kann die Kommunikationseinheit Signale an Beacons übertragen. Aus der relativen Stärke der vom Assistenzsystem ausgesendeten und von mehreren Beacons empfangenen Signale kann in einigen Ausführungsformen eine Position ermittelt werden. Die Ermittlung kann dabei innerhalb des Assistenzsystems (z.B. durch aus den Beacons übermittelten Daten, wie z.B. relative Signalstärke des empfangenen Signals des Assistenzsystems oder der Signallaufzeit) oder innerhalb der Beacons oder eines mit diesen verbundenen Systems stattfinden und dann an das Assistenzsystem übertragen werden, z.B. über die Beacons und/oder durch Verwendung der Kommunikationseinheit.

In einigen Ausführungsformen wird ausschließlich das Mobilfunknetz zur Positionsbestimmung verwendet. In einigen Ausführungsformen wird das Mobilfunknetz zusätzlich zu anderen Verfahren zur Positionsbestimmung verwendet. Dabei kann z.B. die Signalstärke mehrerer Mobilfunkbasisstationen zur Positionsbestimmung verwendet werden. Umgekehrt kann in einigen Ausführungsformen das Assistenzsystem eine Positionsbestimmung durch das Mobilfunknetz veranlassen.

Das erfindungsgemäße Assistenzsystem umfasst in einigen Ausführungsformen eine Satellitennavigationseinheit. Eine Satellitennavigationseinheit umfasst einen Empfänger für Signale, insbesondere elektromagnetische Wellen, insbesondere Hochfrequenz.

Die Satellitennavigationseinheit des Assistenzsystems ist dazu geeignet und/oder konfiguriert, die Signale von Navigationssatellitensystemen zu empfangen und auszuwerten, z.B. autonom oder in Zusammenhang mit dem Prozessor. Das Assistenzsystem kann dabei vorzugsweise Signale von einem oder mehreren der folgenden Navigationssatellitensystemen auswerten: Global Position System (GPS), GLONASS, Galileo und Beidou.

In einigen Ausführungsformen wird die Positionsbestimmung durch die Satellitennavigationseinheit mit mindestens einer weiteren Methode der Positionsbestimmung kombiniert, z.B. durch eine Kombination mit Mobilfunkdaten. Dabei kann in einigen Ausführungsformen die Zeit bis zur ersten Positionsbestimmung, die bei einer ersten Positionsbestimmung durch Satellitennavigation (Fix) erheblich sein kann, reduziert werden. Dies kann beispielsweise durch assisted GPS (A-GPS) geschehen, bei dem GPS-Daten in Verbindung mit Mobilfunkdaten zu einem erheblichen schnelleren Fix führen können. Dadurch kann der Strombedarf des Assistenzsystems in einigen Ausführungsformen erheblich gesenkt werden.

In einigen Ausführungsformen umfasst das erfindungsgemäße Assistenzsystem mindestens einen Sensor. In einigen Ausführungsformen ist der Sensor dazu geeignet und/oder konfiguriert, Parameter des Trägers zu messen und/oder zu überwachen. Derartige Parameter können z.B. Vitalparameter wie z.B. Herzfrequenz, Sauerstoffsättigung von Hämoglobin (SpO2), Atemfrequenz und/oder Blutdruck sein.

In einigen Ausführungsformen können ein oder mehrere der folgenden Parameter von dem Assistenzsystem gemessen und/oder überwacht werden: Glukosekonzentration im Blut, Lungenfunktion (z.B. akustisch), Position, Lage, Beschleunigung und/oder Temperatur. Zudem kann das Assistenzsystem Sensoren umfassen, die eine Exsikkose oder Inkontinenz detektieren. Geeignete Sensoren für die Bestimmung der genannten und weiterer Parameter können in einigen Ausführungsformen sein: optische Sensoren wie z.B. Fotodiode, -transistoren und -widerstände, Pulsoximeter, akustische Sensoren (z.B. Mikrofone), Sensoren für Kraft, Dehnung, Glukose (invasiv oder nicht-invasiv), Inertialmesseinheiten, Beschleunigungsmesser, Gyroskope, Magnetometer, Luftdrucksensoren, Feuchtigkeitssensoren und/oder Berührungssensoren (z.B. mechanische Schalter, optische Sensoren und/oder kapazitive Sensoren).

In einigen Ausführungsformen kann der Sensor über den Tragezustand Auskunft geben, d.h. ob das Assistenzsystem wie vorgesehen vom Träger getragen wird oder gegebenenfalls bereits vom Träger entfernt wurde.

In einigen Ausführungsformen weist das Assistenzsystem mindestens ein Bedienelement (z.B. einen Schalter oder Taster) auf, insbesondere ein Bedienelement, mit dem der Träger manuell einen Hilferuf auslösen kann. Ein derartiger Hilferuf kann dann in einigen Ausführungsformen über das Kommunikationsmodul weitergeleitet werden. Beispielsweise kann eine Sprachverbindung hergestellt werden, so dass der Träger Beschwerden mündlich kommunizieren kann. In diesen und einigen anderen Ausführungsformen weist das Assistenzsystem mindestens ein Mikrofon und/oder mindestens einen Lautsprecher auf. Alternativ oder zusätzlich kann das Assistenzsystem dazu geeignet und/oder konfiguriert sein, ein optisches oder akustisches Warnsignal abzugeben.

Die Bestimmung von Position, Lage und/oder Beschleunigung, z.B. durch Inertialmesseinheiten, Beschleunigungsmesser, Gyroskope und/oder Magnetometer, kann in einigen Ausführungsformen dazu verwendet werden, ein Bewegungsprofil des Trägers aufzuzeichnen und/oder ungewöhnliche und/oder gefährliche Bewegungen oder Lagen zu detektieren.

In einigen Ausführungsformen kann ein zeitabhängiges Bewegungsprofil verwendet werden (z.B. abhängig von Tageszeit und/oder Wochentag).

In einigen Ausführungsformen kann das Assistenzsystem dazu geeignet und/oder konfiguriert sein, ein Bewegungsprofil automatisch zu erstellen und erhebliche Abweichungen zu detektieren, um dann bei derartigen Abweichungen z.B. ein Ereignis auszulösen.

In einigen Ausführungsformen umfasst das Assistenzsystem eine Echtzeituhr.

Durch ein Abweichen von einem zuvor aufgezeichneten Bewegungsprofil kann in einigen Ausführungsformen beispielsweise detektiert werden, wenn sich der Träger aus gewohnter Umgebung entfernt und sich verirrt hat. Ein derartiges Abweichen kann in einigen Ausführungsformen dazu führen, dass eine Positionsbestimmung eingeleitet wird. Umgekehrt kann in einigen Ausführungsformen die Positionsbestimmung ganz oder teilweise deaktiviert bleiben, solange ein aktuelles Bewegungsprofil mit einem vorbestimmten Bewegungsprofil übereinstimmt. Dadurch kann in einigen Ausführungsformen der durchschnittliche Energiebedarf des Assistenzsystems reduziert werden.

In einigen Ausführungsformen kann durch die Verwendung von Sensordaten, z.B. von Inertialmesseinheiten, Beschleunigungsmessern, Gyroskopen und/oder Magnetometern, ein Sturz detektiert werden. Ein Sturz kann ein bestimmtes Bewegungsprofil aufweisen, z.B. kann eine Beschleunigung jenseits eines vorbestimmten Schwellenwertes und/oder eine bestimmte Lage und/oder Lageänderung auf einen Sturz hinweisen. Ein detektierter Sturz kann ein Ereignis im Sinne der Erfindung darstellen, das bestimmte Schritte des Assistenzsystems auslöst.

In bestimmten Ausführungsformen der Erfindung ist das Assistenzsystem mit mindestens einem physisch getrennten Sensor funktionsverbunden. Wenn das Assistenzsystem beispielsweise am Oberkörper getragen wird, so kann ein Pulssensor am Armgelenk befestigt und mit dem Assistenzsystem durch drahtlose Kommunikation verbunden sein.

In einigen Ausführungsformen ist das Assistenzsystem dazu konfiguriert, unmittelbar am Körper getragen zu werden. Dies kann in einigen Ausführungsformen ein Detektieren bestimmter Parameter verbessern. Beispielsweise kann das Assistenzsystem bei direktem Hautkontakt Hauttemperatur, Herzfrequenz und Tragezustand mit hoher Zuverlässigkeit und Genauigkeit detektieren.

In einigen Ausführungsformen ist das Assistenzsystem als ein Kleidungsstück oder ein Verband ausgeführt, beispielsweise als ein Hemd, Unterhemd, eine Weste, ein Socken, ein Schuh oder ein Pflaster, oder weist dergleichen auf. In einigen Ausführungsformen wird das Assistenzsystem durch ein derartiges Kleidungsstück oder einen derartigen Verband am Körper fixiert.

In einigen Ausführungsformen kann das Assistenzsystem Ereignisse feststellen und beim Eintritt eines oder mehrerer Ereignisse weitere Schritte veranlassen. Ein Ereignis im Sinne der Erfindung kann in einigen Ausführungsformen ein detektiertes Fallen sein. In einigen Ausführungsformen kann ein Ereignis in einem Über- und/oder Unterschreiten von Schwellwerten für einen oder mehrere gemessene Parameter bestehen. Beispielsweise kann ein Ereignis in einem Unterschreiten einer Schwelle für eine gemessene Beschleunigung, gemessene Herzfrequenz, für eine gemessene Sauerstoffsättigung von Hämoglobin und/oder eine gemessene Atemfrequenz bestehen. Ein Ereignis kann auch das Entfernen des Assistenzsystems vom Körper oder ein niedriger Ladezustand des Akkus sein.

Zudem kann auch eine mangelnde Bewegung über eine gewisse Zeit ein Ereignis darstellen, dies kann beispielsweise einer Schmerzerkennung, Dekubitusprophylaxe und/oder Sturzprophylaxe dienen.

Die Positionsbestimmung durch Kommunikationseinheit und/oder Satellitennavigationseinheit kann in bestimmten Ausführungsformen im Rahmen eines Geofencing durchgeführt werden. Dabei kann das Verlassen eines zuvor definierten Gebietes ein Ereignis darstellen (z.B. Verlassen eines bestimmten Gebäudes oder Verlassen eines bestimmten Geländes).

Ein Ereignis kann weiterhin darstellen, dass die Messwerte eines oder mehrerer Sensoren außerhalb eines plausiblen Wertebereichs liegen oder mehrere Sensoren in Kombination keine plausiblen Messwerte bereitstellen. Ein derartiges Ereignis kann z.B. auf einen Defekt des Geräts, eine zu niedrige Versorgungsspannung oder ein Entfernen des Assistenzsystems vom Körper hinweisen.

Ein eingetretenes Ereignis kann in einigen Ausführungsformen weitere Schritte auslösen, beispielsweise Informationen versenden. In einigen Ausführungsformen umfassen die ausgelösten Schritte einen oder mehrere der folgenden Schritte:
- Ermitteln der Position mittels Kommunikationseinheit und/oder Satellitennavigationseinheit;
- Benachrichtigen einer Zentrale (z.B. einem Server), eines Notdienstes oder einer Hilfsperson (z.B. Notarzt, Sanitäter, Angehöriger oder Nachbar), beispielsweise über Textnachricht im GSM-System (SMS) oder über eine Datenübertragung im mobilen Internet (z.B. über Mobilfunk, WiFi oder Bluetooth), z.B. an ein Mobilgerät wie ein Telefon oder ein Tablet; und
- Aussenden eines akustischen und/oder optischen Alarmsignals.

In einigen Ausführungsformen ist das Assistenzsystem teilweise oder komplett wasserdicht verschlossen. In einigen Ausführungsformen ist das Assistenzsystem oder Teil von diesem derart konfiguriert, dass ein Desinfizieren z.B. mit herkömmlichen Haut- und/oder Flächendesinfektionsmitteln (z.B. Isopropanol) möglich ist, ohne das Assistenzsystem zu beschädigen. Dazu kann das Assistenzsystem, oder Teile davon, mit einer wasserundurchlässigen Masse verschlossen sein, beispielsweise mit Kunstharz oder silikonhaltigen Materialien.

In einigen Ausführungsformen umfasst das Assistenzsystem zusätzlich zu einem am Träger zu tragenden mobilen Teil einen oder mehrere stationäre Teile, vorzugsweise eine oder mehrere stationäre Kommunikationseinheiten. Eine derartige Kommunikationseinheit kann beispielsweise ein Beacon sein, der Funksignale aussenden kann. Das Assistenzsystem ist in einigen Ausführungsformen dazu geeignet, mittels der stationären Kommunikationseinheit eine Positionsbestimmung des mobilen Teils durchzuführen. Dazu kann beispielsweise ein Beacon periodisch und/oder auf einen Auslöser hin eine eindeutige Kennung versenden. Alternativ oder zusätzlich kann das Assistenzsystem eine Femtozelle umfassen, das heißt eine Basisstation für ein Mobilfunknetz mit geringer Reichweite, die beispielsweise dazu eingesetzt werden kann, den Empfang in einem Gebäude, in dem sich der Träger häufig aufhält, zu verbessern. Durch einen besseren Empfang kann ein am Körper getragenes Mobilfunkmodul gegebenenfalls mit geringerem Energiebedarf betrieben werden sowie vermieden werden, dass ein Ereignis aufgrund eines Funklochs nicht übermittelt werden kann.

In einigen Ausführungsformen umfasst das Assistenzsystem weitere oder andere Sensoren, die insbesondere solche mit den folgenden Sensorfunktionen sein können:
- Mikrophone, insbesondere konfiguriert zur Interpretation von Atemgeräuschen (Atemfrequenz, Atemtiefe, Aspiration, etwa zur Diagnose von Bronchitiden, Pneumonien oder dergleichen) und/oder von Magen- und Darmgeräuschen (etwa zur Diagnose von Obstipation);
- Reflektorischer SpO2-Sensor für Sauerstoffsättigung
- Kapazitives EKG (etwa zur Bestimmung der Herzfrequenz oder Erregungsleitung);
- Sensor zur Bestimmung des Flüssigkeitsstatus oder zur Flüssigkeitsversorgung (Dehydrierung, Exsikkose, Volumendepletion);
- Thermometer zur Temperaturmessung.

In einigen Ausführungsformen umfasst das Assistenzsystem ein oder mehrere Gehäuse oder Teilgehäuse, in welchen einzelne Komponenten des Assistenzsystems untergebracht sind.

In einigen Ausführungsformen stehen ein oder mehrere der vorstehend genannten Gehäuse oder Teilgehäuse mittels wenigstens eines Verbindungsabschnitts miteinander in Verbindung. Verbindungsabschnitte können beispielsweise - optional von Signalleitern abgesehen - frei von Komponenten des Assistenzsystems sein. Sie können optional vorzugweise zur elastischen, plastischen, gelenkigen oder dergleichen Verbindung zwischen benachbarten oder mittels Verbindungsabschnitt verbundenen Gehäusen oder Teilgehäusen, in welchen einzelne Komponenten des Assistenzsystems unterbracht sind, dienen. Mittels dieser Ausgestaltung ist es dem Assistenzsystem möglich, sich in gewissem Umfang an die Oberflächenanatomie seines Trägers anzupassen. Dies mag vor allem dann als angenehm oder vorteilhaft empfunden werden, wenn der Träger, beispielsweise beim Schlafen, auf dem in der Hauptauflage steckenden Assistenzsystem zum Liegen kommt.

Ein vergleichbarer, vorteilhafter Effekt wird in einigen Ausführungsformen dadurch erreicht, dass erste und zweite Abschnitte wenigstens eines Gehäuses oder Teilgehäuses erste bzw. zweite, voneinander verschiedene Biegeeigenschaften aufweisen. Dies kann wiederum durch Gelenkabschnitte, die Wahl von verschiedenen Materialien und/oder unterschiedlichen Materialdicken, Geometrieunterschiede oder dergleichen des Gehäuses oder Teilgehäuses erzielt werden. Eine solche Ausgestaltung kann auch für die hierin genannten Biegeabschnitte vorgesehen sein.

Manche oder alle erfindungsgemäßen Ausführungsformen können einen, mehrere oder alle der oben und/oder im Folgenden genannten Vorteile aufweisen.

Ein erzielbarer Vorteil mancher Ausführungsformen kann darin bestehen, dass die Hautauflage nicht jedes Mal gewechselt werden muss, wenn Komponenten, die mittels der Hautauflage am Körper des Trägers gehalten werden, während der erforderlichen Tragedauer der konkreten Hautauflage oder während der Dauer einer Behandlung des Trägers ausgetauscht werden müssen.

So kann beispielsweise eine Stromversorgungseinheit einer medizinischen oder nicht-medizinischen Einheit, die mittels Hautauflage über Tage am Körper gehalten wird, und die ausgetauscht werden muss, bevor die Hautauflage an sich abgenommen oder ausgetauscht gehört, aus dem Assistenzsystem entnommen und ersetzt werden. Die Hautauflage kann folglich bis auf weiteres am Körper verbleiben. Reizungen der Haut durch häufiges Wechseln der Hautauflage, wie sie beim Wechseln eines aufgeklebten Wundpflasters bekannt sind, können somit verringert oder gar vermieden werden.

Die vorliegende Erfindung wird im Folgenden anhand der beigefügten Zeichnung, in welcher gleiche Bezugszeichen gleiche oder ähnliche Bauteile bezeichnen, exemplarisch erläutert. In den Figuren gilt:
- Fig. 1: zeigt eine erfindungsgemäße Hautauflage in einer ersten exemplarischen Ausführungsform;
- Fig. 2: zeigt eine erfindungsgemäße Hautauflage in einer zweiten exemplarischen Ausführungsform in Draufsicht;
- Fig. 3: zeigt die aus Fig. 2 bekannte Hautauflage mit Blick auf eine Seite der Hautauflage im Teilschnitt;
- Fig. 4: zeigt eine schematische Darstellung eines Assistenzsystems;
- Fig. 5: zeigt eine Ausführungsform des Assistenzsystems;
- Fig. 6: zeigt eine weitere Darstellung eines Assistenzsystems;
- Fig. 7: zeigt eine Anordnung von Beacons;
- Fig. 8a,b: zeigen eine erfindungsgemäße Hautauflage mit einem Assistenzsystem in einer dritten exemplarischen Ausführungsform in verschiedenen Ansichten;
- Fig. 9a: zeigt die dritte exemplarische Ausführungsform, aufgeklebt auf dem Rücken einer Person;
- Fig. 10a,b,c: zeigen eine erfindungsgemäße Hautauflage mit einem Assistenzsystem in einer vierten exemplarischen Ausführungsform in verschiedenen Ansichten;
- Fig. 11a,b: zeigen eine erfindungsgemäße Hautauflage mit einem Assistenzsystem in einer fünften exemplarischen Ausführungsform in verschiedenen Ansichten;
- Fig. 12a,b: zeigen eine erfindungsgemäße Hautauflage mit einem Assistenzsystem in einer sechsten exemplarischen Ausführungsform in verschiedenen Ansichten; und
- Fig. 13a,b: zeigen die dritte, vierte, fünfte und sechste Ausführungsform in einem direkten Vergleich nebeneinander angeordnet.

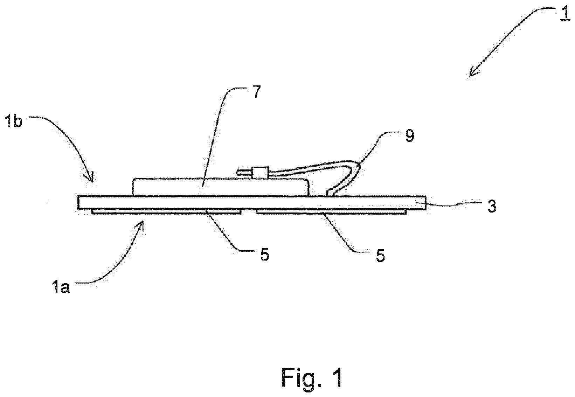

**Fig. 1** zeigt eine erste Ausführungsform der erfindungsgemäßen Hautauflage 1. Diese weist ein flächiges Trägermaterial 3 auf. Hautauflage 1 und/oder Trägermaterial 3 weisen eine im Gebrauch der Haut eines Trägers der Hautauflage 1 zugewandte Seite 1a und eine der Haut im Gebrauch abgewandte Seite 1b auf.

Auf der der Haut zugewandten Seite 1a weist das Trägermaterial 3 Mittel zum Verbinden der Hautauflage 1 mit der Haut auf. Diese Mittel können adhäsiv sein, etwa in Form einer oder mehrerer Klebeschichten 5 oder Klebeabschnitte.

Auf der der Haut abgewandten Seite 1b weist das Trägermaterial 3 wenigstens einen ersten Taschenabschnitt 7 auf oder ist mit diesem - direkt oder indirekt - verbunden.

Der erste Taschenabschnitt 7 ist angeordnet, um von der der Haut abgewandten Seite 1b her zugänglich zu sein.

Der erste Taschenabschnitt 7 weist einen Aufnahmeabschnitt auf, der optional mittels eines Verschlussmittels 9 verschlossen oder verschließbar ist. Das Verschlussmittel 9 ist in Fig. 1 exemplarisch als Verschlusslasche gezeigt. Andere Verschlüsse, etwa Reißverschluss, Klettverschluss, Klebeverschluss, Magnetverschluss und dergleichen, können alternativ oder ergänzend vorgesehen sein und sind von der vorliegenden Erfindung umfasst.

Der erste Taschenabschnitt 7 kann beherbergt das gesamte Assistenzsystem. Dasselbe gilt für die weiteren, im Folgenden erläuterten Taschenabschnitte, Kammern und dergleichen.

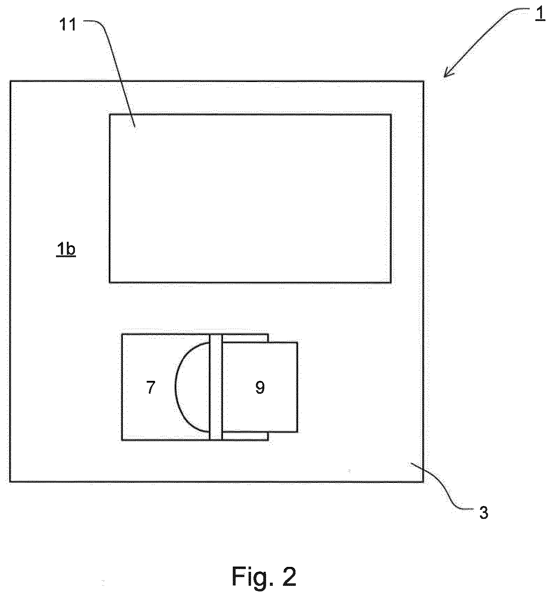

**Fig. 2** zeigt eine erfindungsgemäße Hautauflage 1 in einer zweiten exemplarischen Ausführungsform in Draufsicht.

Die Hautauflage 1 der Fig. 2 entspricht im Wesentlichen jener der Fig. 1. Ergänzend zu jener der Fig. 1 weist sie jedoch eine zusätzliche Kammer 11 auf. Mittels der Kombination aus erstem Taschenabschnitt 7 und zusätzlicher Kammer 11 ist es vorteilhaft möglich, unterschiedliche Gegenstände in der Hautauflage 1 unterzubringen und sie gemeinsam mit dieser auf der Haut des Benutzers zu deponieren.

Die Trennung zwischen erstem Taschenabschnitt 7 und zusätzlicher Kammer 11 erlaubt es, verschiedene Gegenstände am Benutzer vorzusehen und dabei getrennte Zugänge zu diesen vorzusehen. So kann es sein, dass, je nach Verwendung der Hautauflage 1, die Hautauflage 1 einerseits Gegenstände aufnehmen soll, zu denen der Zugang vergleichsweise häufig nötig ist, und andererseits Gegenstände, zu denen der Zugang vergleichsweise selten erforderlich ist, während die Hautauflage 1 getragen wird. Die verwendeten Verschlussmittel 9, die für den ersten Taschenabschnitt 7 und für die zusätzliche Kammer 11 gewählt werden, können dies widerspiegeln bzw. entsprechend ausgewählt sein. So können die Anforderungen an die Handhabung bei Aufnahmen, zu denen selten bis nie Zugang erforderlich ist, geringer sein als im umgekehrten Fall. Entsprechend kostengünstig können die jeweiligen Verschlussmittel ausgewählt sein.

In Fig. 2 sind der erste Taschenabschnitt 7 und die zusätzliche Kammer 11 ohne räumliche Verbindung miteinander gezeigt. Dies kann alternativ anders sein. So kann ein Kanal, Tunnel oder dergleichen das Lumen des ersten Taschenabschnitts 7 und das Lumen der zusätzlichen Kammer 11 miteinander verbinden.

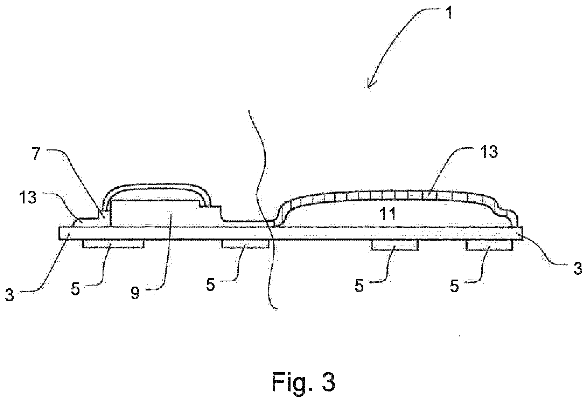

**Fig. 3** zeigt die Hautauflage 1 der Fig. 2 mit Blick auf deren Seite aus einer Rechts-Links-Richtung bezogen auf die Ausrichtung in Fig. 2. In einem rechts in Fig. 3 gelegenen Bereich der Hautauflage 1 ist ein Abdeckmaterial 13 geschnitten dargestellt.

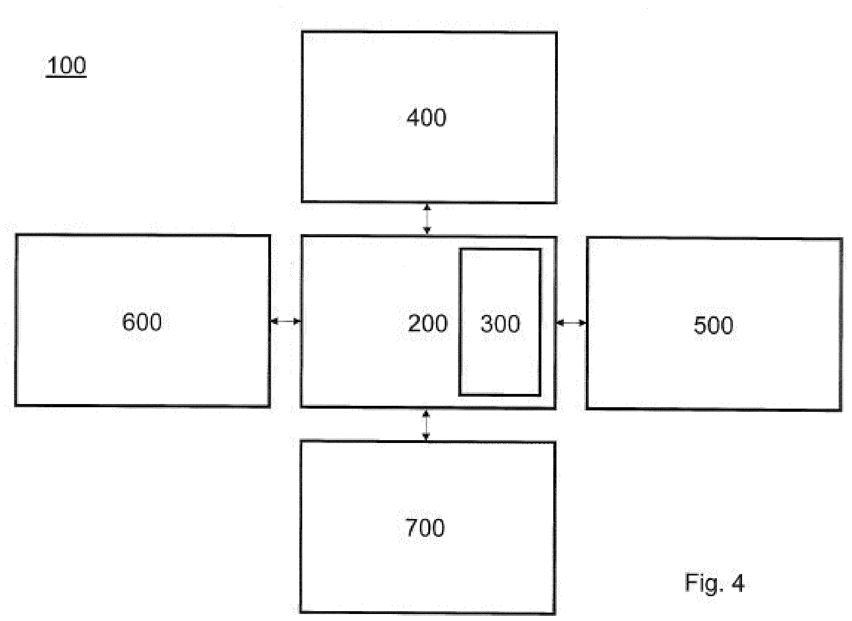

**Fig. 4** zeigt eine schematische Darstellung eines Assistenzsystems 100, das als eine Hautauflage 1 ausgeführt ist. Die technischen Bauteile der Hautauflage 1 umfassen einen Prozessor 200 mit Speicher 300 sowie eine Kommunikationseinheit 400 und eine Stromversorgungseinheit 500.

In der in Figur 4 gezeigten Ausführungsform umfasst das Assistenzsystem 100 zudem eine optionale Satellitennavigationseinheit 600 und mindestens einen optionalen Sensor 700.

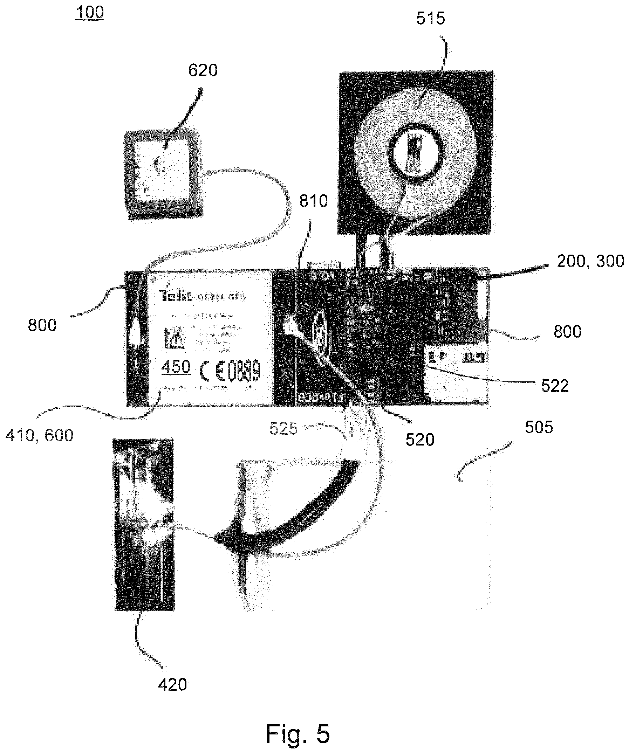

Auch in der Darstellung eines Assistenzsystems 100 in **Fig. 5** ist der Prozessor 200 mit Speicher 300 gezeigt.

Die Stromversorgungseinheit 500 umfasst einen Lithium-Ionen-Akku 505 mit einer nominalen Spannung von 3,7 V und einer Kapazität (Ladungsmenge) von mindestens 1500 mAh, was in der Regel für einen Batteriebetrieb des Assistenzsystems 100 über mindestens 24 Stunden ausreicht. Darüber hinaus umfasst die Stromversorgungseinheit 500 eine optionale induktive Ladeeinrichtung 510, die es ermöglicht, den Akku 505 kabellos zu laden. Dazu umfasst die Stromversorgungseinheit 500 eine Empfangsspule 515 und Ladeelektronik 520 gemäß dem Standard Qi. Zudem enthält die Stromversorgungseinheit zwei DC/DC-Wandler 522 für 3,3V und 1,5V Ausgangsspannung (für I/O bzw. Prozessorkern). Um eine möglichst durchgehende Energieversorgung des Assistenzsystems 100 zu ermöglichen, kann der Akku 505 des Assistenzsystems 100 ausgetauscht werden. Dazu ist der Akku 505 über einen Steckverbinder 525 mit der Stromversorgungseinheit 500 verbunden. Ein entladener Akku 505 kann so durch einen geladenen Akku 505 ersetzt werden und der entladene Akku 505 z.B. außerhalb des Assistenzsystems 100 wiederaufgeladen werden.

Induktive Ladegeräte, die das Assistenzsystem 100 drahtlos mit Ladestrom versorgen können, können in Möbel, Kissen oder Textilien, beispielsweise in Sessel, Stühle, Kissenbezüge, Betttücher oder Matratzenschoner, eingearbeitet sein, so dass das Assistenzsystem 100 im gewöhnlichen Tagesablauf des Trägers aufgeladen werden kann, ohne das Assistenzsystem 100 vom Körper zu entfernen.

Eine Baugruppe des Assistenzsystems 100 enthält z.B. ein Infineon MD8710 System-on-a-Chip mit einem ARM Cortex-R4-Prozessor 200 und ein Bluetooth-Modul 480. Eine weitere Baugruppe enthält ein Mobilfunkmodul 410 und eine Satellitennavigationseinheit 600, die hier als ein kombiniertes Modul mit Kommunikationseinheit und Satellitennavigationseinheit 450, nämlich als ein GSM/GPS-Modul 450 (z.B. Telit GE-864GPS) ausgeführt sind, das sowohl zur Mobilfunkkommunikation (einschließlich TCP/IP-Datentransfer) als auch für assisted GPS genutzt werden kann.

Bis auf die Antenne für Satellitennavigation 620 (ausgeführt insbesondere als Antenne für GPS 620) und die Antenne für Mobilfunk 420 sind alle Baugruppen auf mehreren mehrlagigen Platinen 800 montiert (hier auf zwei 6-lagigen Platinen 800 mit einer Größe von jeweils etwa 35 mm x 30 mm), die mit einem flexiblen Film 810 mit Leiterbahnen verbunden sind (hier ein flexibler Polyamidfilm, der sich über den Verbindungsbereich in die beiden Platinen 800 fortsetzt und dort die Lagen 3 und 4 bildet).

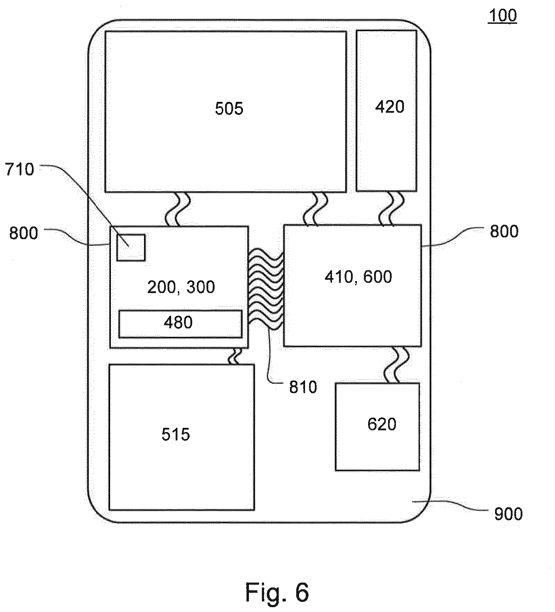

**Fig. 6** zeigt eine weitere Ausführungsform des Assistenzsystems 100, wobei die unterschiedlichen Baugruppen auf einer flexiblen reißfesten Fasermatte 900 befestigt und in ein Elastomer eingebracht sind, so dass das Assistenzsystem 100 dauerhaft, widerstandsfähig, wasserdicht und desinfizierbar ist.

Das in Fig. 6 gezeigte Assistenzsystem 100 umfasst einen Akku 505, der einen Prozessor 200 mit Speicher 300 versorgt, sowie ein GPS/GSM-Modul 450, das eine Kombination des Mobilfunkmoduls 410 und der Satellitennavigationseinheit 600 darstellt. Ein Modul für Kurzstreckenfunkkommunikation 480, hier ein Bluetooth-Modul 480, ist in Funktionseinheit mit dem Prozessor 200 ausgeführt. Alternativ kann ein derartiges Modul zur Kurzstreckenfunkkommunikation 480 getrennt vom Prozessor 200 ausgeführt sein, zum Beispiel zusammen mit dem Mobilfunkmodul 410. Mit der Satellitennavigationseinheit 600 ist eine aktive Antenne für GPS 620 und mit dem Mobilfunkmodul 410 eine Antenne für Mobilfunk 420 verbunden. Die Platine 800, auf der der Prozessor 200 mit Speicher 300 befestigt ist, ist mit der Platine 800, auf der das Mobilfunkmodul 410 und die Satellitennavigationseinheit 600 befestigt sind, über einen flexiblen Film 810, der Leiterbahnen aufweist, funktionsverbunden.

Ein derartig kompaktes Assistenzsystem 100 kann mit Hilfe einer Hautauflage 1, insbesondere eines Pflasters, beispielsweise am Rücken eines Trägers befestigt werden.

Um eine erfolgreiche Positionsbestimmung innerhalb von Gebäuden durchführen zu können, kann das Assistenzsystem 100 elektromagnetische Wellen von Beacons 950 empfangen, d.h. von Sendern, die periodisch eine eindeutige Kennung übermitteln. Derartige Beacons 950 können z. B. Bluetooth verwenden, um eine eindeutige Kennung zu versenden, z.B. ihre MAC-Adresse und gegebenenfalls zusätzlich einen eindeutig identifizierbaren Namen. Das Assistenzsystem 100 ist zum Empfang der Beacons 950 beispielsweise mit einem Bluetooth-Modul 480 ausgestattet. Das Bluetooth-Modul 480 kann Teil einer Satellitennavigationseinheit 600 und/oder einer Kommunikationseinheit 400 sein oder getrennt davon angeordnet sein, z.B. zusammen mit dem Prozessor 200.

Das in Fig. 6 dargestellte Assistenzsystem 100 weist einen oder mehrere Sensoren 700 auf. Die Sensoren 700 in Fig. 6 umfassen einen Beschleunigungssensor (z.B. ein digitaler Drei-Achsen-Beschleunigungssensor wie der ADXL345 von Analog Devices), ein Gyroskop und optional ein Magnetometer, die in Kombination eine inertiale Messeinheit (inertial measurement unit, IMU 710) darstellen. Eine derartige IMU 710 kann dazu verwendet werden, ein Ereignis zu detektieren. Ein derartiges durch die IMU 710 detektierbares Ereignis kann beispielsweise ein Sturz des Trägers sein. Dabei können eine hohe Beschleunigung, ein Lagewechsel von Stehen zu Liegen und/oder eine hohe Winkelgeschwindigkeit für ein Sturzereignis sprechen. Für eine Sturzdetektion können beispielsweise geometrische Mittel aller Beschleunigungssensorachsen des letzten Datensatzes bestimmt werden. Falls die berechneten geometrischen Mittel eine vorbestimmte Schwelle überschreiten, wird dies in einem ersten Teil als ein möglicher Sturz gewertet. Um die falsch positiv detektierten Sturzereignisse zu vermindern, wird als optionaler zweiter Teil die statische Komponente eines Lagewechsels (Transition von Sitzen oder Stehen zu Liegen) und/oder die dynamische Komponente des Lagewechsels aus Gyroskopdaten überprüft. Mit Hilfe der IMU 710 können auch bestimmte Bewegungsabläufe, wie z. B. Gehen, Rennen oder Radfahren, bestimmt werden und gegebenenfalls über die Kommunikationseinheit 400 übermittelt werden.

Weitere durch die IMU 710 detektierbare Ereignisse können ein Aufstehen aus dem Bett in der Nacht, ein Umherirren (Gehbewegungen mit zahlreichen Richtungsänderungen) oder eine ungewöhnlich geringe Bewegungsaktivität sein. Zudem hat das Assistenzsystem 100 in Fig. 6 einen optionalen Luftdrucksensor, der dazu verwendet werden kann, Höhenveränderungen des Trägers zu detektieren. Mit Hilfe des Luftdrucksensors kann beispielsweise bestimmt werden, ob der Träger sich von einem in ein anderes Stockwerk bewegt.

In den Ausführungsformen, in denen das Assistenzsystem 100 am Rumpf des Trägers befestigt ist (z.B. mit einer Hautauflage 1), kann eine Lagebestimmung (z. B. Stehen vs. Liegen) häufig besonders zuverlässig ausgeführt werden, da die IMU 710 sich nicht relativ zum Rumpf bewegen kann und daher eindeutige Lageinformationen des Rumpfs aus der IMU 710 bestimmt werden können.

Der Energiebedarf des Assistenzsystems 100 hängt stark vom Aktivierungszustand der verschiedenen Teilsysteme ab. So ist in einigen Ausführungsformen die Verwendung der IMU 710 sehr sparsam, das Bluetooth-Modul 480 weist einen geringen Energiebedarf auf, wohingegen GSM-Modul 410 und Satellitennavigationseinheit 600 einen hohen Energiebedarf aufweisen. Daher ist es wichtig, die Kommunikationseinheit 400, insbesondere das GSM-Modul 410, sowie die Satellitennavigationseinheit 600 möglichst lange ausgeschaltet zu lassen. Dazu wird in einigen Ausführungsformen Kommunikationseinheit 400, GSM-Modul 410 und/oder Satellitennavigationseinheit 600 nur auf ein bestimmtes Ereignis hin aktiviert, beispielsweise nach einem detektierten Sturz oder einem fehlenden Empfang von Signalen eines Beacons 950 über eine vorbestimmte Zeit.

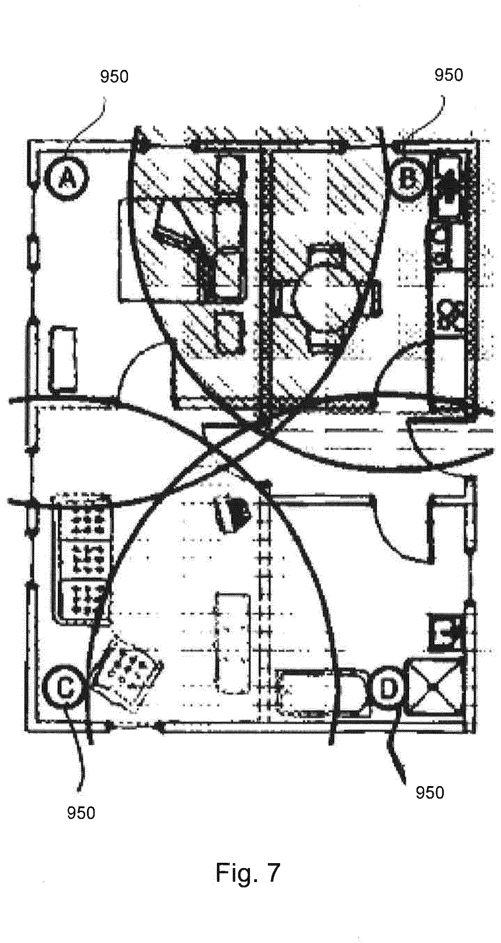

**Fig. 7** zeigt die beispielhafte Anordnung von vier Bluetooth-Beacons 950 (A-D) in vier Räumen einer Wohnung. Die Reichweite der Beacons 950 ist jeweils als ein Kreisbogen dargestellt. Wie aus der Fig. 7 zu sehen ist, überschneiden sich Empfangsbereiche der Beacons 950 teilweise. Aus dem empfangenen Signal kann daher auf einen Bereich geschlossen werden, in dem sich das Assistenzsystem 100 befindet. Dabei kann auch die Empfangsstärke der einzelnen Beacons 950 berücksichtigt werden (z. B als received signal strength indicator RSSI). Im Assistenzsystem 100 können beispielsweise die MAC-Adressen und Positionen von bekannten Beacons 950 abgespeichert sein, um eine Positionsbestimmung bereits innerhalb des Assistenzsystems 100 vorzunehmen. Alternativ können Beacons 950 in einigen Ausführungsformen ihre Position zusammen mit einer eindeutigen Kennung senden. Beacons 950 können auch in größeren Gebäuden verwendet werden, beispielsweise in den Räumen eines Krankenhauses.

Um den Energiebedarf zu reduzieren, kann das Assistenzsystem 100 so konfiguriert sein, dass beim Empfang der Signale eines Beacons 950 die Satellitennavigationseinheit 600 deaktiviert wird, da bereits durch die Beacons 950 eine Positionsbestimmung möglich ist und die Satellitennavigationseinheit 600 in der Regel einen wesentlich höheren Energiebedarf als ein Modul zur Kurzstreckenfunkkommunikation 480, z. B. ein Bluetooth-Modul 480, hat. Das Assistenzsystem 100 kann derart konfiguriert sein, dass bei einem fehlenden Empfang von Signalen eines Beacons 950 ein Alarm ausgelöst wird, bei dem beispielsweise ein Server oder eine Zentrale darüber informiert wird, dass sich der Träger mit einer gewissen Wahrscheinlichkeit außerhalb der Reichweite der Beacons 950 aufhält. Alternativ oder zusätzlich kann in diesen Fällen das GSM-Modul 410 und/oder das GPS-Modul 600 aktiviert werden, um eine Standortbestimmung auch ohne Beacons 950 zu ermöglichen. Zudem kann ein Alarm ausgelöst werden, wenn ein Signal eines bestimmten Beacons 950 empfangen wird, z. B. weil sich der Träger in einem falschen Bereich befindet.

In einigen Fällen kann das Assistenzsystem 100 die Beacons 950 umfassen. Dann kann beispielsweise das Assistenzsystem 100 einen mobilen Teil, der am Körper zu tragen ist, und zudem einen stationären Teil aufweisen, der ein oder mehrere Beacons 950 umfasst. Alternativ oder zusätzlich kann das Assistenzsystem 100 eine Zentrale umfassen, beispielsweise einen Server, der mit dem mobilen Teil des Assistenzsystems 100 interagiert und auf vorbestimmte Ereignisse hin Informationen an bestimmte Adressen weiterleitet, beispielsweise einen Notruf auslöst.

Ein Beacon 950, wie er in Fig. 7 dargestellt ist, kann beispielsweise in einem Steckergehäuse untergebracht sein, so dass der Beacon 950 in der Wohnung des Trägers leicht zu installieren und unauffällig ist.

Wenn sich der Träger im Freien befindet, dann kann das Assistenzsystem 100 eine Positionsbestimmung mittels assisted GPS durchführen, was unter Umständen schneller und/oder mit niedrigerem Energiebedarf möglich ist. Dabei werden Hilfsinformationen aus dem Mobilfunknetz verwendet. Auch WiFi-Daten können für eine schnellere Positionsbestimmung herangezogen werden, wenn z. B. MAC-Adressen von WiFi-Adaptern mit ihren Positionen in einer Datenbank verzeichnet sind. Eine derartige Datenbank kann für relevante Gebiete in dem Assistenzsystem 100 gespeichert sein oder von dem Assistenzsystem 100 von einem Server abgerufen werden.

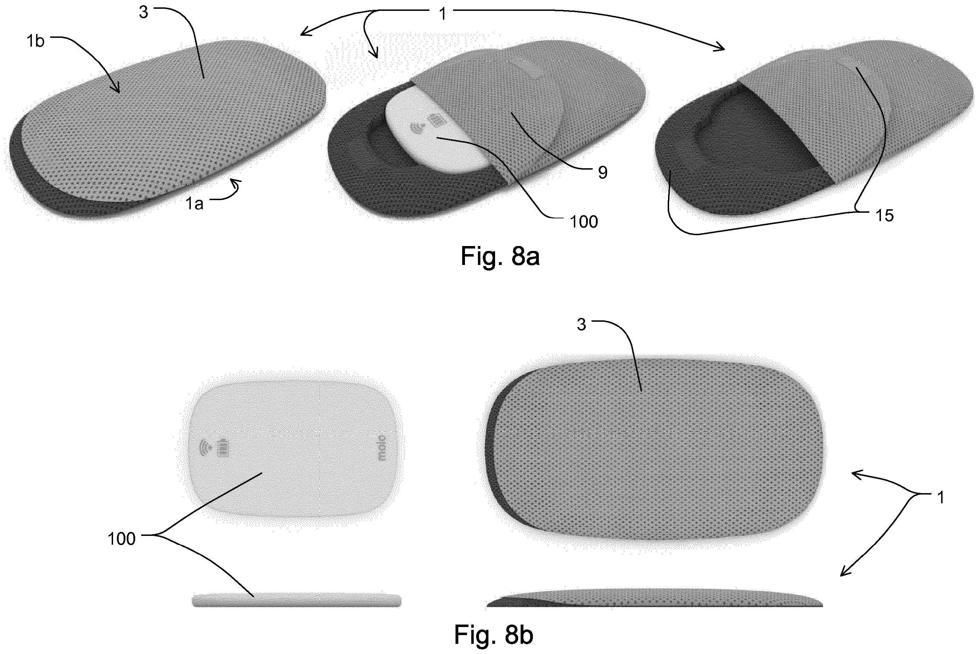

**Fig. 8a** zeigt eine erfindungsgemäße Hautauflage 1 mit einem Assistenzsystem 100 in einer dritten exemplarischen Ausführungsform in einer perspektivischen Ansicht. Die rechte Abbildung der Fig. 8a zeigt die geöffnete Hautauflage 1, das heißt, die Verschlusslasche 9 ist aufgeklappt. In dieser Anordnung kann das Assistenzsystem 100 in die Hautauflage 1 eingeschoben werden.

Die Anordnung mit eingeschobenen Assistenzsystem 100 zeigt die mittlere Abbildung der Fig. 8a. In der linken Abbildung der Fig. 8a ist die Verschlusslasche 9 zugeklappt und bedeckt oder verdeckt das zuvor eingeschobene Assistenzsystem 100. Die Hautauflage 1 weist eine im Gebrauch der Haut eines Trägers der Hautauflage 1 zugewandte Seite 1a und eine der Haut im Gebrauch abgewandte Seite 1b auf.

Optional weist die Hautauflage 1, vorzugsweise auf seiner Oberseite und/oder in seinem Trägermaterial 3 oder einem Querschnitt hiervon wenigstens eine Vertiefung als Positionierhilfe, Aufnahme oder Begrenzung für das Assistenzsystem 100 auf.

Mittels der Vertiefung kann das Assistenzsystem 100 z. B. mittig in der Hautauflage 1 oder in/auf dem Trägermaterial 3 positioniert werden. Damit kann vorteilhaft ein Verrutschen oder Verschieben des Assistenzsystems 100 innerhalb der Hautauflage 1 und/oder innerhalb des Trägermaterials 3 verhindert werden.

Ergänzend oder alternativ kann eine Vertiefung das Assistenzsystem 100 innerhalb der Hautauflage 1 einbetten, vorzugsweise so, dass ebene Abschlussflächen oder Übergänge zwischen Oberseite des Trägermaterials und der Oberseite des Assistenzsystems 100 entstehen. Mögliche Kanten und Ecken können damit vermieden werden, und der Tragekomfort für den Träger kann vorteilhaft erhöht werden.

In der Ausführungsform der Fig. 8a weist die Verschlusslasche 9 und das entsprechende Gegenstück des Trägermaterials 3 (alternativ des Abdeckmaterials 13) einen magnetischen Verschluss 15 auf.

Der magnetische Verschluss 15 kann auf verschiedene Weisen ausgeführt sein, beispielsweise mittels eines Magneten an oder in der Verschlusslasche 15 und einem ferromagnetischen Material auf der Gegenseite (also am Trägermaterial 3 oder am Abdeckmaterial 13), oder umgekehrt, oder Magnete beidseitig.

Der Magnet und/oder das ferromagnetische Material können identische oder unterschiedliche Geometrien und Ausführungsformen aufweisen. Sie können beispielsweise knopfförmig, bandförmig, gekapselt, beschichtet, ummantelt usw. sein.

Ein magnetischer Verschluss 15 ist ein vorteilhaft einfacher, komfortabler und nutzerfreundlicher Verschluss, der sich leicht öffnen und schließen lässt. Zudem kann er nahezu beliebig dünn ausgestaltet sein. Er trägt daher nicht oder vergleichsweise nur wenig in der Höhe auf.

Die Hautauflage 1 weist auf der der Haut zugewandten Seite 1a Mittel zum Verbinden der Hautauflage 1 mit der Haut auf, die hier nicht weiter beschrieben werden (siehe beispielsweise Beschreibung zur Fig. 1).

Das Material der Hautauflage 1 und des Trägermaterials 3 kann eine sogenannte Unterfütterung aufweisen, beispielsweise in Form einer Polsterung, die als Schaumstoff ausgeführt sein kann.

Die Materialien der Hautauflage 1 insgesamt und/oder nur des Trägermaterials 3 oder des Abdeckmaterials 13 können luftdurchlässig ("atmungsaktiv") und/oder feuchtigkeitsdurchlässig (für einen Abtransport von Schweißbildung) sein. In den Fig. 8a und 8b sind die Materialien zumindest an ihren Oberflächen offenporig oder mit Durchgangsöffnungen dargestellt, wobei die Poren ganz oder teilweise die Materialien durchdringen können.

**Fig. 8b** zeigt rechts die erfindungsgemäße Hautauflage 1 mit dem Assistenzsystem 100 der Fig. 8a in einer Draufsicht (oben) und in einer Seitenansicht (unten).

**Fig. 8b** zeigt links das erfindungsgemäße Assistenzsystem 100 der Fig. 8a in einer Draufsicht (oben) und in einer Seitenansicht (unten).

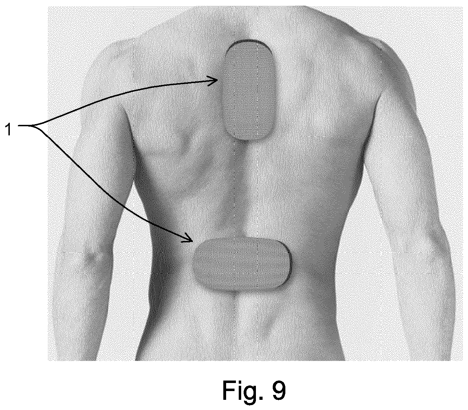

**Fig. 9** zeigt zwei Exemplare der dritten exemplarischen Ausführungsform der erfindungsgemäßen Hautauflage 1 aus den Figuren 8a und 8b mit einem darin eingeschobenen Assistenzsystem 100, welche auf dem Rücken einer Person, optional und rein exemplarisch an zwei verschiedenen Positionen, aufgeklebt ist.

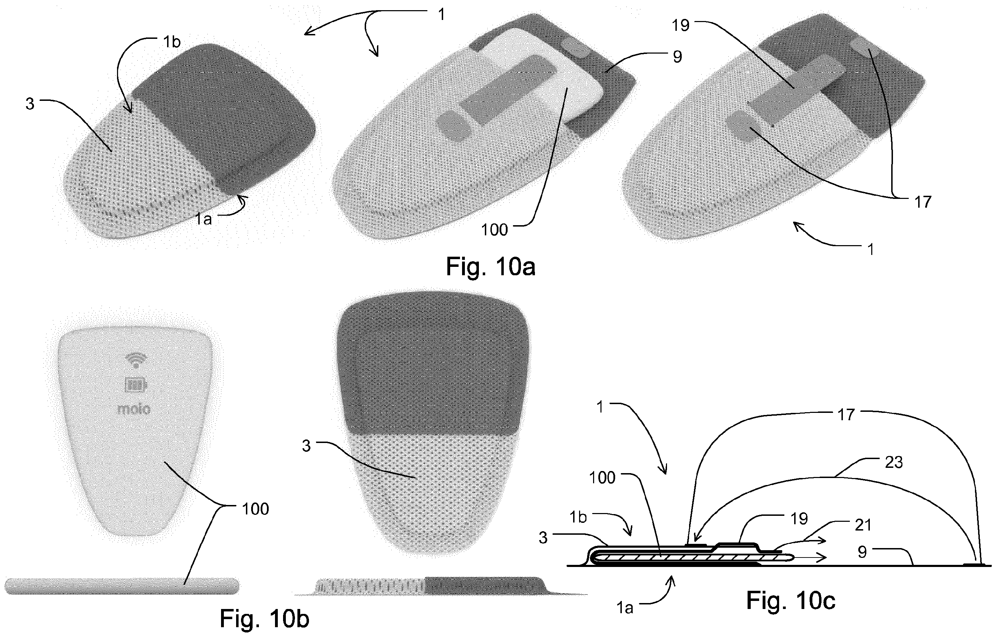

**Fig. 10a** zeigt eine erfindungsgemäße Hautauflage 1 mit einem Assistenzsystem 100 in einer vierten exemplarischen Ausführungsform in einer perspektivischen Ansicht. Gegenüber der dritten Ausführungsform aus den Fig. 8a und 8b ist die Form der Hautauflage 1 und des Assistenzsystems 100 etwas verändert. Die leicht keilförmige Gestalt kann beispielsweise hinsichtlich der anatomischen Anpassung an eine Auflagefläche am Rücken (siehe Fig. 9) vorteilhaft sein.

Weiterhin ist ein Mechanismus, der auch in anderen Ausführungsformen Verwendung finden kann, zum Fixieren der Verschlusslasche 9 am Trägermaterial 3 dargestellt. Dieser Mechanismus kann wie in Fig. 8a und Fig. 8b mittels eines magnetischen Verschlusses 15, mittels eines Klettverschlusses 17 oder in einer anderen Art und Weise ausgeführt sein.

In den Fig. 10a bis 10c ist zusätzlich eine sogenannte Entnahmehilfe zum Herausziehen des Assistenzsystems 100 aus der Hautauflage 1 dargestellt. Die Entnahmehilfe ist rein optional als Schlaufe 19 ausgeführt, deren Funktionsweise zu Fig. 10c erläutert wird.

Die Lasche kann in der Richtung, in welcher das Assistenzsystem 100 in die Hautauflage 1 eingesteckt wird, verlaufen. Sie kann um das entfernte Ende des Assistenzsystems 100 geführt sein und somit zu beiden Seiten (Ober- und Unterseite, oder Teilen hiervon) des Assistenzsystems 100 verlaufen, diese dort optional auch berühren.

Optional kann die Verschlusslasche 9 anstatt wie in den Fig. 10a und Fig. 10b gezeigt nicht nach oben (also zur Schlaufe 19 hin) umgeklappt werden, sondern wie in den Fig. 8a und 8b ausgeführt, nach unten. Damit kann vorteilhaft ein Überlappen oder Übereinanderliegen der Verschlusslasche 9 und des Trägermaterials 3 verhindert werden. Ein Überlappen oder Übereinanderliegen könnte sich für den Träger nachteilig auswirken, da dies zu einer Zunahme der Dicke der gesamten Hautauflage 1 führen könnte, was insbesondere beim Liegen vom Träger als unangenehm und ggf. auf dem Rücken zu möglichen Druckstellen führen könnte.

**Fig. 10b** zeigt die erfindungsgemäße Hautauflage 1 mit dem Assistenzsystem 100 der Fig. 10a in einer Draufsicht (oben) und in einer Seitenansicht (unten). Zu erkennen ist, dass das Assistenzsystem 100 in einer Draufschau ebenfalls optional keilförmig, trapezoid oder dreieckig sein kann.

**Fig. 10c** zeigt schematisch vereinfacht die Entnahmehilfe zum Herausziehen des Assistenzsystems 100 aus der Hautauflage 1. Die Pfeilrichtung 21 zeigt die Zugrichtung der Schlaufe 19 zum Herausziehen des Assistenzsystems 100. Dadurch ist eine vorteilhaft einfache Entnahme des Assistenzsystems 100, beispielsweise zum Austauschen des gesamten Assistenzsystems 100 oder nur der Batterien oder Akkus, aus der Hautauflage 1 möglich. Bei einem Wiedereinschieben des Assistenzsystems 100 wird die Schlaufe 19 wieder mittels des Assistenzsystems 100 und mit diesem zusammen eingeschoben.

Die Pfeilrichtung 23 zeigt die Klapprichtung der Verschlusslasche 9 zum Fixieren der Verschlusslasche 9 auf dem Trägermaterial 3.

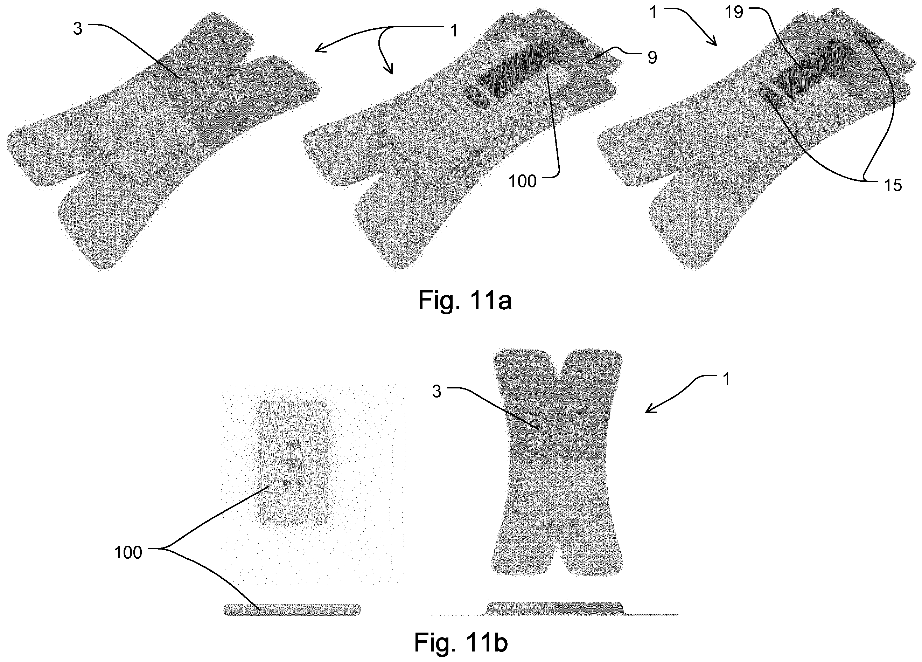

**Fig. 11a,b** zeigen analog zu den Figuren 8a,b und 10a,b,c eine weitere erfindungsgemäße Hautauflage 1 mit einem Assistenzsystem 100 in einer fünften exemplarischen Ausführungsform in verschiedenen Ansichten.

Die Hautauflage 1 ist in dieser exemplarischen oder jeden anderen Ausführungsform entlang wenigstens eines seiner Ränder eingeschnitten, gekerbt, verjüngend, unterteilt oder dergleichen ausgestaltet.

Eine solche Gestalt der Hautauflage 1 kann beispielsweise hinsichtlich der anatomischen Anpassung an eine Auflagefläche am Rücken vorteilhaft sein.

Optional kann die Verschlusslasche 9 anstatt wie in den Fig. 11a und 11b gezeigt nicht nach oben (also zur Schlaufe 19 hin) umgeklappt werden, sondern wie in den Fig. 8a und 8b ausgeführt, nach unten.

Alternativ kann die Verschlusslasche 9 oder jede andere Verschlusseinrichtung Teil des Abdeckmaterials 13 sein. Ist dies der Fall, so kann die Dicke des Sets aus Hautauflage 1 mit eingeschobenem Assistenzsystem 100 vorteilhaft gering gehalten werden.

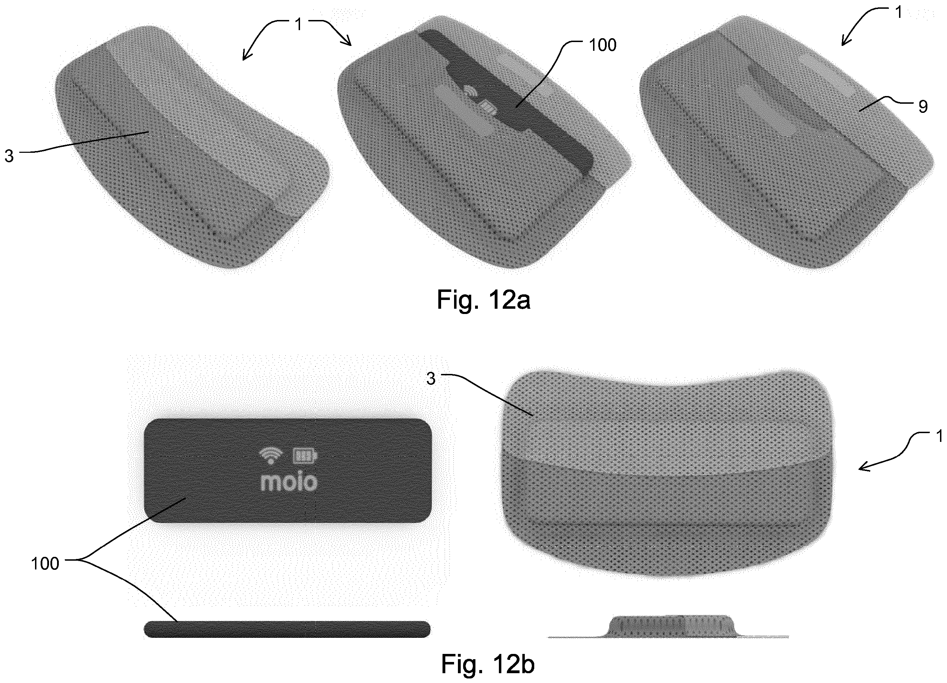

**Fig. 12a,b** zeigen analog zu den Figuren 8a,b, zu den 10a,b,c sowie zu den Figuren 11a,b eine weitere erfindungsgemäße Hautauflage 1 mit einem Assistenzsystem 100 in einer sechsten exemplarischen Ausführungsform in verschiedenen Ansichten.

Die breite Gestalt der Hautauflage 1 und des Assistenzsystems 100 kann beispielsweise hinsichtlich der anatomischen Anpassung an eine Auflagefläche insbesondere im unteren Rückenbereich vorteilhaft sein.

Optional kann die Verschlusslasche 9 anstatt wie in den Fig.12a und 12b gezeigt nicht nach oben umgeklappt werden, sondern wie in den Fig. 8a und 8b ausgeführt, nach unten.

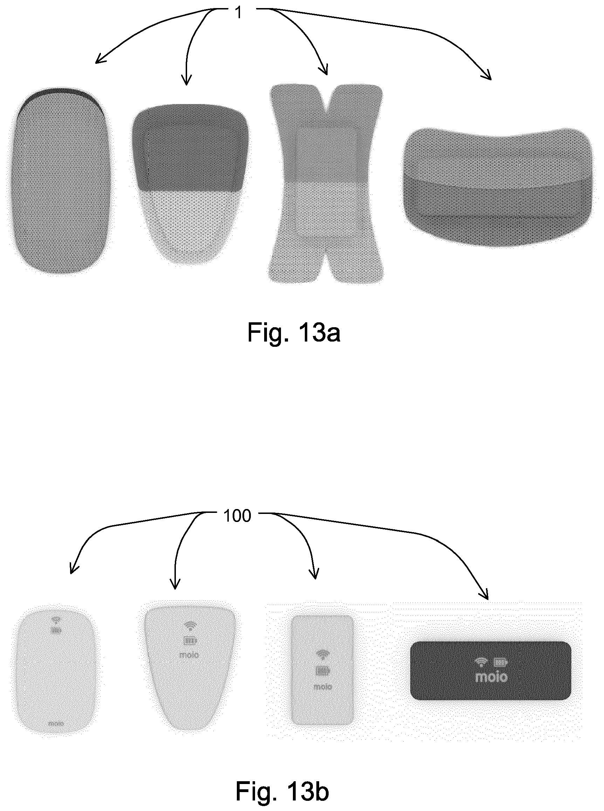

**Fig. 13a,b** zeigen die dritte, vierte, fünfte und sechste Ausführungsform in einem direkten Vergleich jeweils nebeneinander angeordnet.

Zusätzlich kann jede erfindungsgemäße Hautauflage 1 und/oder jedes erfindungsgemäße Assistenzsystem 100 eine Abschirmung gegen elektromagnetische Strahlung, insbesondere auf der körperzugewandten Seite 1a der Hautauflagen 1 (siehe Fig. 8a oder Fig. 10a) aufweisen. Die elektromagnetische Strahlung, die es abzuschirmen oder abzuschwächen gilt, kann vom Assistenzsystem 100 emittiert werden. Die hier angesprochenen Abschirmungen können beispielsweise als Abschirmgitter, metallbedampfte Plastikfolien, Aluminiumfolie-kaschiertes Papier, Graphit- und Leitlack-Schichten oder als andere Abschirmvorrichtungen ausgeführt sein oder dergleichen aufweisen.

Erfindungsgemäße Assistenzsysteme 100 können rein exemplarisch eine Länge von ca. zwischen 4 cm und 12 cm und eine Breite von ca. 2 cm und 10 cm aufweisen. Das Assistenzsystem 100 kann in seiner Länge ein Mindestmaß von 4 cm aufweisen. Das Assistenzsystem 100 kann in seiner Länge ein Höchstmaß von 12 cm aufweisen. Das Assistenzsystem 100 kann in seiner Breite ein Mindestmaß von 2 cm aufweisen. Das Assistenzsystem 100 kann in seiner Breite ein Höchstmaß von 10 cm aufweisen.

Erfindungsgemäße Hautauflagen 1 können rein exemplarisch eine Länge von ca. zwischen 6 cm und 20 cm und eine Breite von ca**.** 4 cm und 15 cm aufweisen. Die Hautauflage 1 kann in ihrer Länge ein Mindestmaß von 6 cm aufweisen. Die Hautauflage 1 kann in ihrer Länge ein Höchstmaß von 20 cm aufweisen.

Die Hautauflage 1 kann in ihrer Breite ein Mindestmaß von 4 cm aufweisen. Die Hautauflage 1 kann in ihrer Breite ein Höchstmaß von 15 cm aufweisen.

### Bezugszeichenliste

- 1: Hautauflage
- 1a: der Haut eines Trägers der Hautauflage zugewandte Seite
- 1b: der Haut eines Trägers der Hautauflage abgewandte Seite
- 3: Trägermaterial
- 5: Klebeschicht
- 7: erster Taschenabschnitt
- 9: Verschlussmittel, Verschlusslasche
- 11: zusätzliche Kammer
- 13: Abdeckmaterial
- 15: magnetischer Verschluss
- 17: Klettverschluss
- 19: Schlaufe
- 21: Zugrichtung der Schlaufe
- 23: Klapprichtung der Verschlusslasche

- 100: Assistenzsystem
- 200: Prozessor
- 300: Speicher
- 400: Kommunikationseinheit
- 410: Mobilfunkmodul, GSM-Modul
- 420: Antenne für Mobilfunk, GSM-Antenne
- 450: kombiniertes Modul mit Kommunikationseinheit und Satellitennavigationseinheit, GSM/GPS-Modul
- 480: Modul für Kurzstreckenfunkkommunikation, Bluetooth-Modul
- 500: Stromversorgungseinheit
- 505: Akku, Lithium-Ionen-Akku
- 510: induktive Ladeeinrichtung
- 515: Empfangsspule
- 520: Ladeelektronik
- 522: DC/DC-Wandler
- 525: Steckverbinder
- 600: Satellitennavigationseinheit, GPS-Modul
- 620: (aktive) Antenne für Satellitennavigation, Antenne für GPS
- 700: Sensor
- 710: inertiale Messeinheit, inertial measurement unit, IMU
- 800: Platine, mehrlagige Platine
- 810: flexibler Film
- 900: Fasermatte
- 950: Beacons

## Patentansprüche

1. System umfassend eine Hautauflage (1) und ein Assistenzsystem (100), wobei die Hautauflage (1) mit einem flächigen Trägermaterial (3) ausgestattet ist, mit einer im Gebrauch der Haut eines Trägers der Hautauflage (1) zugewandten Seite (1a) und einer der Haut abgewandten Seite (1b), wobei das Trägermaterial (3) auf der der Haut zugewandten Seite (1a) zumindest bereichsweise mit einer Klebeschicht (5) versehen ist zum Fixieren der Hautauflage (1) auf der Haut des Trägers, **dadurch gekennzeichnet, dass** die Hautauflage (1) wenigstens einen ersten Taschenabschnitt (7) aufweist, welcher von der der Haut abgewandten Seite (1b) zugänglich ist, wobei der erste Taschenabschnitt das Assistenzsystem (100) aufweist,
wobei das Assistenzsystem (100) mindestens eine/n
- Prozessor (200);
- Speicher (300);
- Kommunikationseinheit (400) umfassend eine Mobilfunkeinheit; und
- Stromversorgungseinheit (500) umfasst.

2. System nach einem der vorangehenden Ansprüche, **dadurch gekennzeichnet, dass** wenigstens ein Element aus der Gruppe bestehend aus erstem Taschenabschnitt (7), zweitem Taschenabschnitt und zusätzlicher Kammer (11) an seiner Unterseite vom Trägermaterial (3) und an seiner Oberseite von einem Abdeckmaterial (13), insbesondere gemeinsam, begrenzt ist.

3. System nach Anspruch 2, **dadurch gekennzeichnet, dass** das Abdeckmaterial (13), beispielsweise mittels einer Klebeschicht (5), eines Klettabschnitts, eines Nahtabschnitts, mit der der Haut abgewandten Seite (1b) des Trägermaterials (3) verbunden ist.

4. System nach Anspruch 2 oder 3, **dadurch gekennzeichnet, dass** das Material des Abdeckmaterials (13) dem Material des Trägermaterials (3) entspricht.

5. System nach einem der vorangehenden Ansprüche, **dadurch gekennzeichnet, dass** wenigstens ein Element aus der Gruppe bestehend aus erstem Taschenabschnitt (7), zweitem Taschenabschnitt und zusätzlicher Kammer (11) ein Verschlussmittel (9) aufweist.

6. System nach einem der vorangehenden Ansprüche, wobei das Assistenzsystem (100) eine Satellitennavigationseinheit (600) umfasst.

7. System nach einem der vorangehenden Ansprüche, wobei das Assistenzsystem (100) mit mindestens einem Sensor (700) ausgestattet ist.

8. System nach einem der vorangehenden Ansprüche, wobei das Assistenzsystem (100) dazu geeignet und/oder konfiguriert ist, am Körper getragen zu werden.

9. System nach einem der vorangehenden Ansprüche, wobei das Assistenzsystem (100) dazu geeignet und/oder konfiguriert ist, ein Ereignis festzustellen.

10. System nach einem der vorangehenden Ansprüche, wobei das Assistenzsystem (100) dazu geeignet und/oder konfiguriert ist, auf ein bestimmtes Ereignis hin weitere Schritte zu veranlassen.

11. System nach einem der vorangehenden Ansprüche, wobei das Assistenzsystem (100) wasserdicht verschlossen ist und/oder desinfizierbar ist.

## Claims

1. A system comprising a dermal patch (1) and an assistance system (100), wherein the dermal patch (1) is equipped with a flat carrier material (3), having, in use, one side (1a) facing the skin and one side (1b) opposite the skin of a wearer of the dermal patch (1), wherein the carrier material (3) on the side (1a) facing the skin is provided at least partially with an adhesive layer (5) for fixing the dermal patch (1) to the skin of the wearer, **characterized in that** the dermal patch (1) comprises at least one first pocket section (7), which is accessible from the side (1b) opposite the skin, wherein the first pocket section comprises the assistance system (100),
wherein the assistance system (100) includes at least one:
- processor (200);
- storage (300);
- communication unit (400) including a mobile radio communication unit;
and
- power supply unit (500).

2. The system according to anyone of the preceding claims, **characterized in that** at least one element from the group consisting of the first pocket section (7), the second pocket section and an additional chamber (11) is delimited on its underside by the carrier material (3) and on its upper side by a covering material (13), in particular jointly.

3. The system according to claim 2, **characterized in that** the covering material (13) is connected to the side (1b) of the carrier material (3) opposite the skin, for example by means of an adhesive layer (5), a Velcro section, a seam section.

4. The system according to claim 2 or 3, **characterized in that** the material of the covering material (13) corresponds to the material of the carrier material (3).

5. The system according to anyone of the preceding claims, **characterized in that** at least one element from the group consisting of the first pocket section (7), the second pocket section and an additional chamber (11) comprises a closure means (9).

6. The system according to anyone of the preceding claims, wherein the assistance system (100) includes a satellite navigation unit (600).

7. The system according to anyone of the preceding claims, wherein the assistance system (100) is equipped with at least one sensor (700).

8. The system according to anyone of the preceding claims, wherein the assistance system (100) is suitable and/or configured to be worn on the body.

9. The system according to anyone of the preceding claims, wherein the assistance system (100) is suitable and/or configured to detect an event.

10. The system according to anyone of the preceding claims, wherein the assistance system (100) is suitable and/or configured to initiate further steps in response to a specific event.

11. The system according to anyone of the preceding claims, wherein the assistance system (100) is sealed watertight and/or can be disinfected.

## Revendications

1. Un système comprenant un revêtement cutané (1) ainsi qu'un système d'assistance (100), où le revêtement cutané (1) est équipé d'un matériau de support plat (3), ayant, à l'usage, un côté (1a) face à la peau et un côté (1b) opposé à la peau, à l'usage d'un porteur du revêtement cutané (1), où le matériau de support (3) sur le côté (1a) face à la peau est pourvu au moins partiellement d'une couche adhésive (5) permettant de fixer le revêtement cutané (1) sur la peau du porteur, **caractérisé en ce que** le revêtement cutané (1) comporte au moins un premier compartiment (7) accessible depuis le côté (1b) opposé à la peau, où le premier compartiment comprend le système d'assistance (100),
où le système d'assistance (100) comprend au moins:
- un processeur (200);
- une mémoire (300);
- une unité de communication (400) comprenant une unité de radiocommunication mobile;
ainsi
- qu'un bloc d'alimentation électrique (500).

2. Le système selon l'une quelconque des revendications précédentes, **caractérisé en ce qu'**au moins un élément du groupe constitué du premier compartiment (7), du second compartiment ainsi que d'une chambre supplémentaire (11) est délimité sur sa face inférieure par le matériau de support (3) et sur sa face supérieure par un matériau de recouvrement (13), notamment conjointement.

3. Le système selon la revendication 2, **caractérisé en ce que** le matériau de recouvrement (13) est relié au côté (1b) du matériau de support (3) opposé à la peau, par exemple au moyen d'une couche adhésive (5), d'une partie Velcro, d'une partie cousue.

4. Le système selon la revendication 2 ou 3, **caractérisé en ce que** le matériau du matériau de recouvrement (13) correspond au matériau du matériau de support (3).

5. Le système selon l'une quelconque des revendications précédentes, **caractérisé en ce qu'**au moins un élément du groupe constitué du premier compartiment (7), du second compartiment ainsi que d'une chambre supplémentaire (11) comprend un moyen de fermeture (9).

6. Le système selon l'une quelconque des revendications précédentes, où le système d'assistance (100) comporte une unité de navigation par satellite (600).

7. Le système selon l'une quelconque des revendications précédentes, où le système d'assistance (100) est équipé d'au moins un capteur (700).

8. Le système selon l'une quelconque des revendications précédentes, où le système d'assistance (100) est adapté et/ou configuré pour être porté sur le corps.

9. Le système selon l'une quelconque des revendications précédentes, où le système d'assistance (100) est adapté et/ou configuré pour détecter un événement.

10. Le système selon l'une quelconque des revendications précédentes, où le système d'assistance (100) est adapté et/ou configuré pour engendrer des étapes supplémentaires en réponse à un événement spécifique.

11. Le système selon l'une quelconque des revendications précédentes, où le système d'assistance (100) est fermé de manière à être étanche à l'eau et/ou peut être désinfecté.
